# EUROPEAN PATENT APPLICATION

(11) **EP 4 786 980 A1**
(43) Date of publication of application: **05.08.2026**
(21) Application number: 24872529.3
(22) Date of filing: 27.09.2024
(51) Int. Cl.: G01N 33/53, G01N 33/531, G01N 33/532, G01N 33/543

(54) **ENHANCER REAGENT FOR CHEMILUMINESCENT REACTION, AND COMPOUND**

(30) Priority: 29.09.2023 JP 2023170516; 30.08.2024 JP 2024148423
(71) Applicant: FUJIFILM Corporation, Tokyo 106-8620 (JP)
(72) Inventor: YOSHIMITSU, Yuji, Kanagawa 2588577 (JP); SUN, Xiaoyin, Kanagawa 2588577 (JP); KAMBARA, Aiko, Kanagawa 2588577 (JP); WATANABE, Kousuke, Kanagawa 2588577 (JP); OGURA, Takahiro, Kanagawa 2588577 (JP); OHIRA, Shino, Kanagawa 2588577 (JP); TERADA, Daisuke, Kanagawa 2588577 (JP); OUCHI, Ryo, Kanagawa 2588577 (JP)
(74) Representative: HGF
(86) International application number: PCT/JP2024/034709
(87) International publication number: WO 2025/070748

(57) **Abstract**

An object of the present invention is to provide an enhancer reagent that enhances chemiluminescence intensity and is capable of extending a measurement upper limit while enabling luminescence measurement at a concentration of a measurement lower limit in a measurement of an analyte, and a compound capable of enhancing chemiluminescence intensity. According to the present invention, there is provided an enhancer reagent for a chemiluminescence reaction including a compound represented by Formula (1).

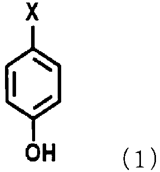

In the formula, X represents a pyridyl group which may be substituted with one or more groups.

## Description

### Technical Field

The present invention relates to an enhancer reagent for a chemiluminescence reaction capable of enhancing chemiluminescence intensity in the measurement of measuring an analyte by using chemiluminescence. The present invention further relates to a compound useful as an enhancer reagent for a chemiluminescence reaction.

### Background Art

In a chemiluminescence measurement system using luminol or the like, it is known that 4-phenylphenol (PPP) and thiazolephenol (TP) are used as an enhancer that enhances chemiluminescence intensity.

Patent Document 1 describes TP (thiazolephenol) as an enhancer, and Patent Document 2 describes PPP, but neither of these refers to a measurement range on a high concentration range side. As an effect, an improvement in sensitivity in a low concentration range is described.

### Prior Art Documents

### Patent Documents

Patent Document 1: JP3030043B
Patent Document 2: JP-H03-005539A

### Summary of Invention

### Object to be solved by the invention

For example, in a measurement of human chorionic gonadotropin (hCG) or estradiol (E2), it is necessary to measure specimens having a wide range of antigen amounts from a low concentration to a high concentration. However, as a result of studies by the present inventors, it has been found that there is a problem that, in a measurement using TP or PPP, it is not possible to measure a specimen having a wide range of antigen amounts, that is, it is not possible to extend a measurement upper limit while maintaining a measurement lower limit and to perform a measurement while maintaining linearity over a wide range.

An object of the present invention is to provide an enhancer reagent that enhances chemiluminescence intensity and is capable of achieving excellent linearity (hereinafter, referred to as a high-end slope) in a high concentration range in a measurement of an analyte. Another object of the present invention is to provide a compound capable of enhancing chemiluminescence intensity.

### Means for solving the object

To achieve the above-described object, the present inventors have made intensive studies and screened various compounds, and as a result, found that an enhancer reagent having a pyridine skeleton can achieve excellent high-end slope. The present invention has been completed based on these findings. That is, according to the present invention, the following inventions are provided.
<1> An enhancer reagent for a chemiluminescence reaction, comprising: a compound represented by Formula (1), 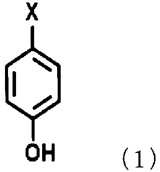 in the formula, X represents a pyridyl group which may be substituted with one or more groups selected from the group consisting of an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a silyl group, a silyloxy group, a hydroxyl group, a cyano group, a nitro group, a halogen atom, a carboxy group, a sulfo group, a phosphonyl group, a phosphoryl group, a boronic acid group, and a sulfanyl group.
<2> The enhancer reagent according to <1>, in which X is a 3-pyridyl group which may be substituted with one or more groups selected from the group consisting of an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a silyl group, a silyloxy group, a hydroxyl group, a cyano group, a nitro group, a halogen atom, a carboxy group, a sulfo group, a phosphonyl group, a phosphoryl group, a boronic acid group, and a sulfanyl group.
<3> The enhancer reagent according to <1>, in which X is a 3-pyridyl group which is substituted with one or more groups selected from the group consisting of an alkyl group, an alkoxy group, a halogen atom, and an amino group.
<4> The enhancer reagent according to <1>, in which X is a 3-pyridyl group which is substituted with one or more groups selected from the group consisting of an alkyl group having 1 to 5 carbon atoms, an alkoxy group having 1 to 5 carbon atoms, and a fluorine atom.
<5> The enhancer reagent according to <1>, in which the compound represented by Formula (1) is any one of the following compounds, 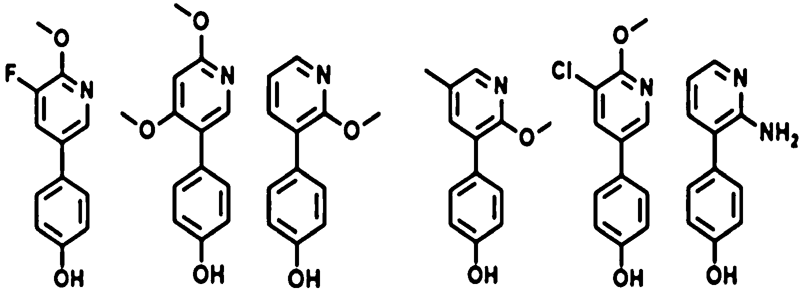 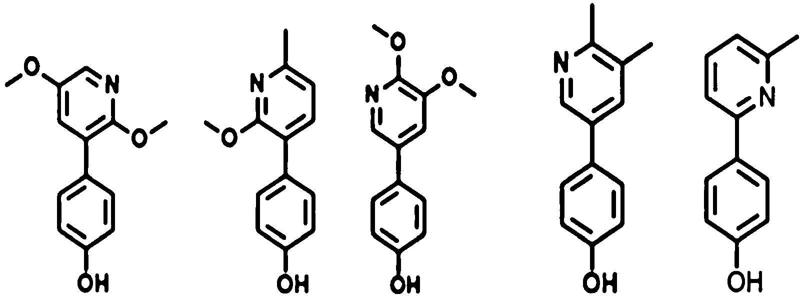 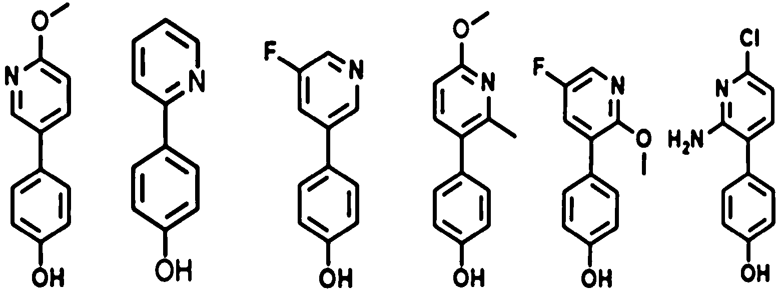 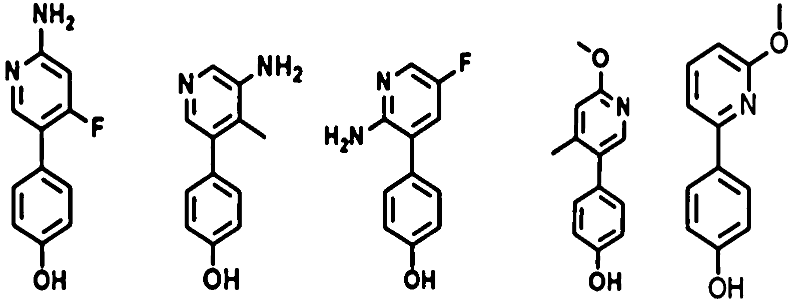 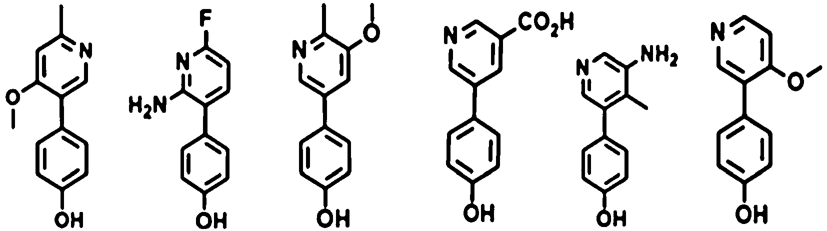 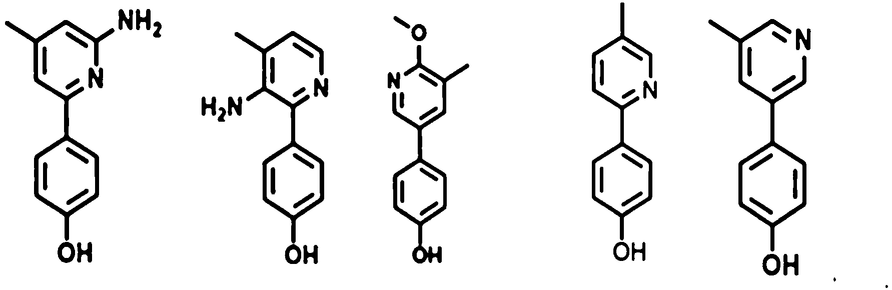
<6> A compound represented by any one of the following formulae,

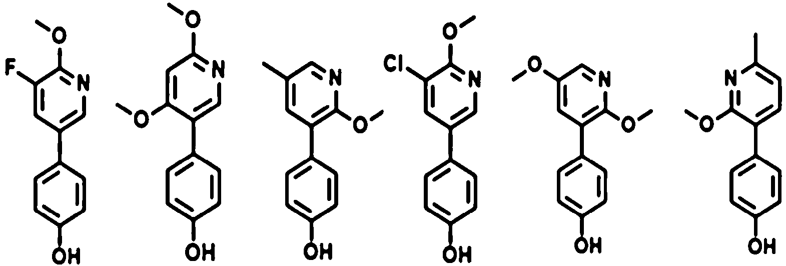

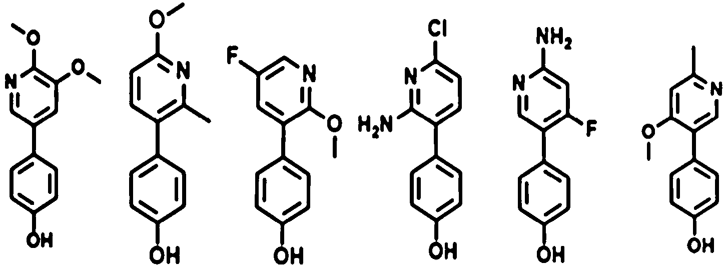

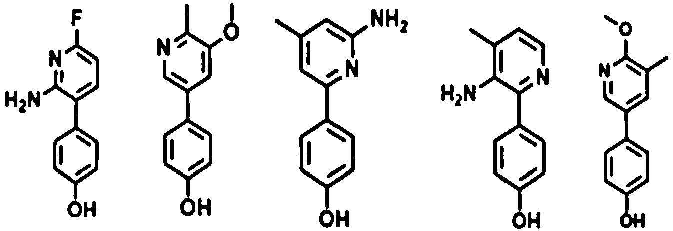

### Effect of the invention

According to the enhancer reagent for a chemiluminescence reaction and the compound according to the present invention, it is possible to achieve excellent high-end slope in a measurement of an analyte.

### Embodiments for carrying out the invention

Hereinafter, the present invention will be described in detail. In the present specification, mM represents mmol/L.

### <Enhancer reagent for chemiluminescence reaction>

The enhancer reagent for a chemiluminescence reaction according to the embodiment of the present invention includes a compound represented by Formula (1). The enhancer reagent for a chemiluminescence reaction is a reagent that can be used to enhance chemiluminescence intensity, increase a luminescence rate, and extend a luminescence time in the measurement of measuring an analyte by using chemiluminescence. 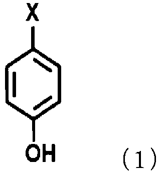 in the formula, X represents a pyridyl group which may be substituted with one or more groups selected from the group consisting of an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a silyl group, a silyloxy group, a hydroxyl group, a cyano group, a nitro group, a halogen atom, a carboxy group, a sulfo group, a phosphonyl group, a phosphoryl group, a boronic acid group, and a sulfanyl group.

X is preferably a pyridyl group which may be substituted with one or more groups selected from the group consisting of an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a silyl group, a silyloxy group, a hydroxyl group, a cyano group, a nitro group, a halogen atom, a carboxy group, a sulfo group, a phosphonyl group, a phosphoryl group, a boronic acid group, and a sulfanyl group.

X is more preferably a 3-pyridyl group which is substituted with one or more groups selected from the group consisting of an alkyl group, an alkoxy group, a halogen atom, and an amino group.

X is still more preferably a 3-pyridyl group which is substituted with one or more groups selected from the group consisting of an alkyl group having 1 to 5 carbon atoms, an alkoxy group having 1 to 5 carbon atoms, and a fluorine atom.

As the alkyl group, an alkyl group having 1 to 5 carbon atoms is preferable. Examples of the alkyl group having 1 to 5 carbon atoms include a methyl group, an ethyl group, a propyl group, a butyl group, and a pentyl group, which may be linear or branched. As the alkyl group having 1 to 5 carbon atoms, a methyl group is preferable.
As the alkenyl group, an alkyl group having 2 to 5 carbon atoms is preferable. Examples of the alkyl group having 2 to 5 carbon atoms include an ethenyl group, a propenyl group, a butenyl group, and a pentenyl group, which may be linear or branched.

As the cycloalkyl group, a cycloalkyl group having 3 to 6 carbon atoms is preferable. Examples of the cycloalkyl group having 3 to 6 carbon atoms include a cyclopropyl group, a cyclobutyl group, a cyclopentyl group, and a cyclohexyl group.

As an aryl group, an aryl group having 6 to 14 carbon atoms is preferable, and examples thereof include a phenyl group, a 1-naphthyl group, a 2-naphthyl group, a biphenylyl group, and a 2-anthryl group.

Examples of the heterocyclic group include a monocyclic or bicyclic or higher heterocyclic group containing one or more atoms selected from an oxygen atom, a nitrogen atom, or a sulfur atom as a heteroatom, and examples thereof include a pyridyl group, a pyrazolyl group, an imidazolyl group, a benzimidazolyl group, a thiazolyl group, an oxazolyl group, a pyrimidyl group, an isoxazolyl group, a triazolyl group, a furanyl group, a thiophenyl group, and a pyrrolyl group.

As the alkoxy group, an alkoxy group having 1 to 5 carbon atoms is preferable. Examples of the alkoxy group having 1 to 5 carbon atoms include a methoxy group, an ethoxy group, a propoxy group, a butoxy group, and a pentyloxy group, which may be linear or branched. As the alkoxy group having 1 to 5 carbon atoms, a methoxy group is preferable.

As the cycloalkoxy group, a cycloalkoxy group having 3 to 6 carbon atoms is preferable. Examples of the cycloalkoxy group having 3 to 6 carbon atoms include a cyclopentyloxy group and a cyclohexyloxy group.

As the aryloxy group, an aryloxy group having 6 to 20 carbon atoms is preferable. Examples of the aryloxy group having 6 to 20 carbon atoms include a phenoxy group.

As the alkoxycarbonyl group, an alkoxycarbonyl group having 2 to 6 carbon atoms is preferable. Examples of the alkoxycarbonyl group having 2 to 6 carbon atoms include a methoxycarbonyl group, an ethoxycarbonyl group, a propoxycarbonyl group, a butoxycarbonyl group, and a pentyloxycarbonyl group.

As the cycloalkoxycarbonyl group, a cycloalkoxycarbonyl group having 4 to 7 carbon atoms is preferable. Examples of the cycloalkoxycarbonyl group having 4 to 7 carbon atoms include a cyclopentyloxycarbonyl group and a cyclohexyloxycarbonyl group.

Examples of the acylamino group include a formylamino group, an alkylcarbonylamino group having 2 to 30 carbon atoms, and an arylcarbonylamino group having 6 to 30 carbon atoms. Specific examples of the acylamino group include a formylamino group, an acetylamino group, a pivaloylamino group, a lauroylamino group, and a benzoylamino group.

Examples of the silyl group include a group represented by -Si(R¹)(R²)(R³). R¹, R², and R³ each independently represent a hydrogen atom or a substituent, and as the substituent, an alkyl group, an alkoxy group, an aryl group, or a heterocyclic group is preferable.

Examples of the silyloxy group include a group represented by -O-Si(R¹)(R²)(R³). R¹, R², and R³ each independently represent a hydrogen atom or a substituent, and as the substituent, an alkyl group, an alkoxy group, an aryl group, or a heterocyclic group is preferable.

Examples of the halogen atom include fluorine, chlorine, bromine, and iodine, and fluorine or chlorine is preferable.

A preferred example of the compound represented by Formula (1) is any one of the following compounds, but the compound represented by Formula (1) is not limited to the following compounds.

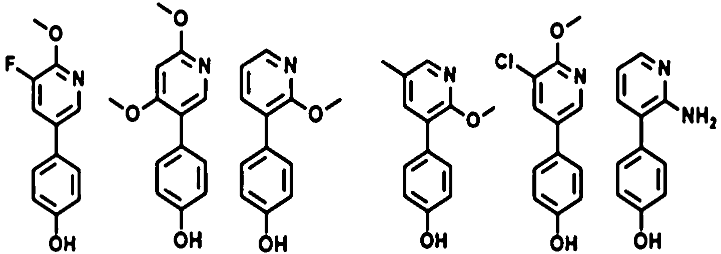

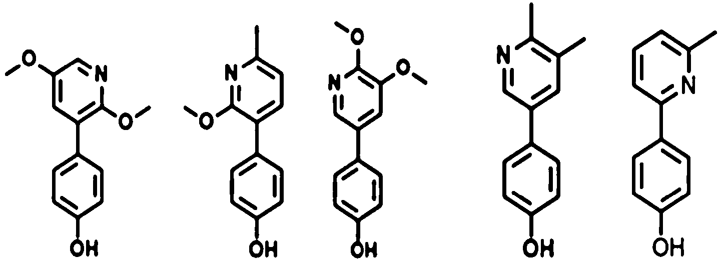

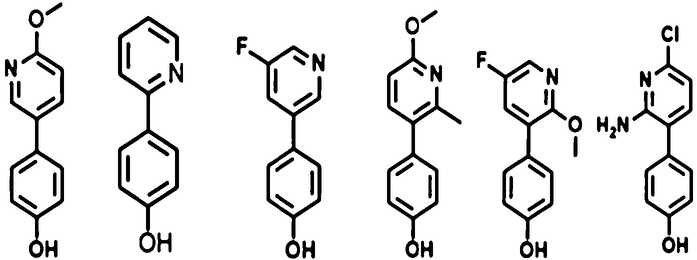

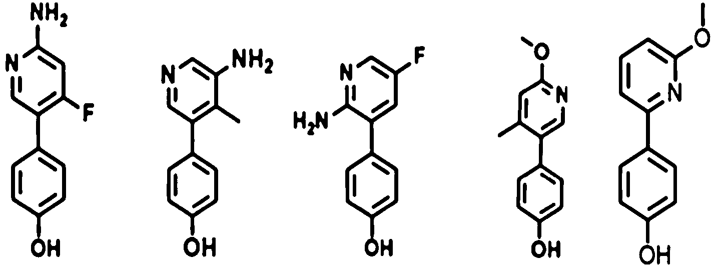

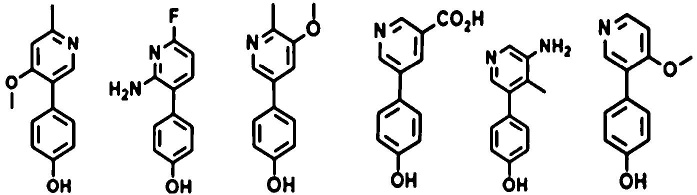

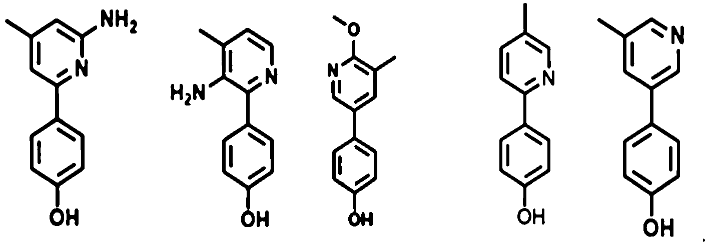

### <Compound>

According to the present invention, the following compound is provided. The following compound is useful as a enhancer reagent for a chemiluminescence reaction.

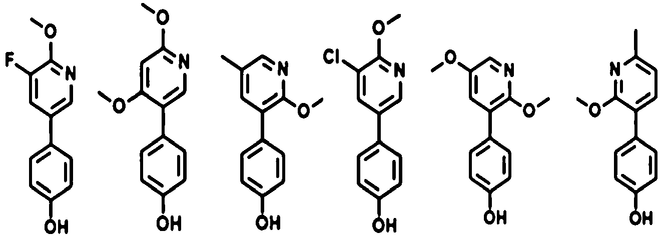

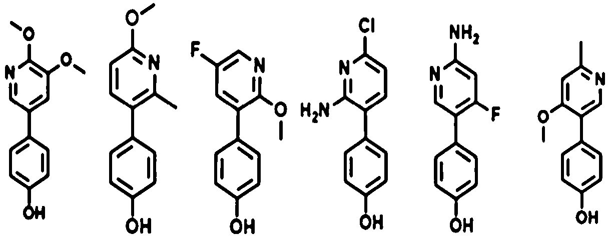

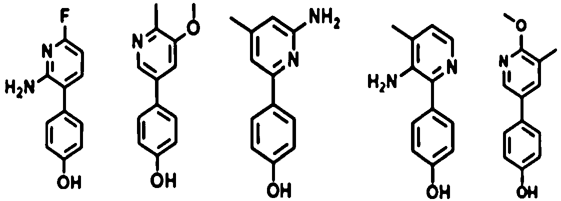

The compound according to the embodiment of the present invention can be synthesized according to the method described in "Synthesis of 4-(5-fluoro-6-methoxypyridin-3-yl)phenol" or "Synthesis of 4-(6-methoxypyridin-2-yl)phenol" in Examples.

### <Measurement method for measurement target substance>

The enhancer reagent for a chemiluminescence reaction according to the embodiment of the present invention can be used for measuring an analyte.

The measurement method for an analyte using the enhancer reagent for a chemiluminescence reaction according to the embodiment of the present invention may be a measurement method for an analyte contained in a test sample.

The measurement method of an analyte using the enhancer reagent for a chemiluminescence reaction according to the embodiment of the present invention is a method including:
(1) an analyte having peroxidase bound to the analyte through a binding substance that recognizes the analyte, or
   an analyte having peroxidase bound to the analyte through a binding substance that recognizes a complex of the analyte and the binding substance that recognizes the analyte;
(2) a chemiluminescent substance; and
(3) a oxidizing agent;
in the presence of one or more compounds represented by Formula (1).

Examples of the test sample include biological samples such as biological body fluids such as serum, blood, blood plasma, and urine; lymph; blood cells; and various cells, but the test sample is not particularly limited.

In the present invention, the analyte contained in the test sample is not particularly limited, and may be any measurement target substance. The analyte may be an antigen.

In the present invention, the analyte is not particularly limited, and examples thereof include all measurement target substances that are considered to be usually measurable by an immunological assay method or the like, such as steroids, peptides, hormones, antibodies, proteins, drugs, metabolites, vitamins, contained in the biological specimen. Specific examples of the endocrine function-related substance include thyroid stimulating hormone (TSH), parathyroid hormone (iPTH), growth hormone (GH), somatomedin C (IGF-1), luteinizing hormone (LH), follicle stimulating hormone (FSH), prolactin (PRL), adrenocorticotropic hormone (ACTH), vasopressin, oxytocin, somatostatin, enkephalin, β-endorphin, thyroxine, triiodothyronine, thyroglobulin, anti-thyroglobulin antibody, anti-T3 antibody, anti-T4 antibody, anti-TSH antibody, calcitonin, catecholamine, dopamine, serotonin, aldosterone, renin, angiotensin, cortisol, deoxycortisol, cortisone, corticosterone, deoxycorticosterone, androsterone, progesterone, pregnenolone, estrogen, estrone, estriol, estradiol, testosterone, human chorionic gonadotropin, insulin, anti-insulin antibody, C-peptide, glucagon, gastrin, secretin, cyclic AMP, cyclic GMP, prostaglandins, thromboxane, erythropoietin, histamine, and the like. Specific examples of the tumor-related substance include CEA, ferritin, β2-microglobulin, elastase, α-fetoprotein, neuron-specific enolase, prostate-specific antigen, CA19-9, and the like. Specific examples of the drug or vitaminrelated substance include phenobarbital, phenytoin, carbamazepine, primidone, ethosuximide, valproic acid, acetazolamide, sultiame, glutethimide, clonaxepam, nitrazepam, diazepam, pentobarbital, secobarbital, bupivacaine, mepivacaine, lidocaine, procainamide, quinidine, digoxin, digitoxin, theophylline, amitriptyline, imipramine, amikacin, gentamicin, tobramycin, cephalexin, sulfamethoxazole, methotrexate, cyclosporine, methylprednisolone, salicylic acid, acetaminophen, indomethacin, allopurinol, vitamin A, carotene, vitamin B1, vitamin B2, vitamin B6, vitamin B12, folic acid, vitamin C, vitamin D, and vitamin E, and the like. Specific examples of the serum or blood plasma protein-related substance include albumin, α1-microglobulin, α1-antitrypsin, α2-macroglobulin, haptoglobin, hemopexin, transferrin, myoglobin, IgG, IgM, IgA, IgD, IgE, fibrinogen, antithrombin, plasminogen, antiplasmin, protein C, rheumatoid factor, anti-DNA antibody, C-reactive protein, and the like. Specific examples of the virus or infectious disease-related substance include HBs antigen, HBs antibody, HBc antibody, HTLV-I antibody, HTLV-III antibody, TPHA, various virus antigens, and various virus antibodies. However, the present invention is not limited thereto.

The concentration of the compound represented by Formula (1) is preferably 10 µmol/L or more and 500 µmol/L or less, more preferably 50 µmol/L or more and 500 µmol/L or less, still more preferably 75 µmol/L or more and 400 µmol/L or less, and particularly preferably 100 µmol/L or more and 300 µmol/L or less.

The concentration referred to herein is a concentration in a reaction solution in the measurement of reacting the analyte having peroxidase bound to the analyte through a binding substance that recognizes the analyte, the chemiluminescent substance, and the oxidizing agent in the presence of one or more compounds represented by Formula (1).

The analyte having peroxidase bound to the analyte through a binding substance that recognizes the analyte, the chemiluminescent substance, and the oxidizing agent are reacted in the presence of one or more compounds represented by Formula (1).

The chemiluminescent substrate is preferably a nitrogen-containing heterocyclic compound having an amino group or a salt thereof, and examples thereof include a 2,3-dihydro-1,4-phthalazinedione compound having an amino group, 8-amino-5-chloro-7-phenylpyrido[3,4-d]pyridazine-1,4(2H,3H)-dione sodium salt (L-012) or a salt thereof, and the like.

The structure of L-012 is shown below.

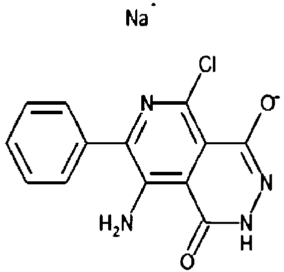

As the 2,3-dihydro-1,4-phthalazinedione compound, for example, known 2,3-dihydro-1,4-phthalazinedione compounds described in JP1990-291299A (JP-H2-291299A), JP1998-319015A (JP-H10-319015A), JP2000-279196A, and the like, and mixtures thereof can be used. Among these, luminol, a luminol derivative (for example, isoluminol, N-aminohexyl-N-ethylisoluminol (AHEI), N-aminobutyl-N-ethylisoluminol (ABEI), and the like), and metal salts thereof (alkali metal salts and the like) are preferable, and luminol and a metal salt thereof are more preferable, and a sodium salt of luminol is particularly preferable.

The concentration of the chemiluminescent substance is preferably 0.1 mmol/L or more and 10 mmol/L or less, more preferably 0.2 mmol/L or more and 7.5 mmol/L or less, still more preferably 0.25 mmol/L or more and 5.0 mmol/L or less, and particularly preferably 0.25 mmol/L or more and 2.5 mmol/L or less.

The concentration referred to herein is a concentration in a reaction solution in the measurement of reacting the analyte having peroxidase bound to the analyte through a binding substance that recognizes the analyte, the chemiluminescent substance, and the oxidizing agent in the presence of one or more compounds represented by Formula (1).

Examples of the oxidizing agent include an aqueous solution of known oxidizing agents described in JP1996-261943A (JP-H8-261943A) and JP2000-279196A, and the like [inorganic peroxides (hydrogen peroxide, sodium perborate, potassium perborate, and the like), organic peroxides (dialkyl peroxides and acyl peroxides, and the like), peroxo acid compounds (peroxosulfuric acid and peroxophosphoric acid, and the like), and the like]. Among these, from the viewpoint of storage stability and the like, hydrogen peroxide, sodium perborate, and potassium perborate are preferable, and hydrogen peroxide is more preferable.

The concentration of the oxidizing agent is appropriately set depending on the type thereof, and a measurement method and measurement conditions to be applied, but from the viewpoint of a chemiluminescence enhancement effect and the like, it is preferably 0.5 mmol/L or more and 20 mmol/L or less, more preferably 1 mmol/L or more and 10 mmol/L or less, and still more preferably 1.4 mmol/L or more and 3.5 mmol/L or less.

The concentration referred to herein is a concentration in a reaction solution in the measurement of reacting the analyte having peroxidase bound to the analyte through a binding substance that recognizes the analyte, the chemiluminescent substance, and the oxidizing agent in the presence of one or more compounds represented by Formula (1).

In an example, the analyte having peroxidase bound to the analyte through a binding substance that recognizes the analyte may be obtained by reacting the analyte with a first binding substance that recognizes the analyte to form a complex, and reacting the complex with a second binding substance that recognizes the analyte, the second binding substance having peroxidase bound to the analyte.

In an example, the analyte having peroxidase bound to the analyte through a binding substance that recognizes a complex of the analyte and a first binding substance that recognizes the analyte may be obtained by reacting the analyte with the first binding substance that recognizes the analyte to form a complex, and reacting the complex with "a third binding substance that recognizes the complex of the analyte and the first binding substance that recognizes the analyte", the third binding substance having peroxidase bound to the analyte.

For example, the analyte in a sample can be measured using a solid-phase carrier such as magnetic silica particles, having a surface on which a first binding substance that recognizes the analyte is immobilized.

Examples of the binding substance that recognizes the analyte (the first binding substance that recognizes the analyte or the second binding substance that recognizes the analyte) include substances that bind to the analyte by mutual reactions such as an "antigen"-"antibody" reaction, a "glycan"-"protein" reaction, a "glycan"-"lectin" reaction, a "protein"-"peptide chain" reaction, a "protein"-"protein" reaction, and a "protein"-"nucleotide chain" reaction.

Examples of the binding substance that recognizes the complex include substances that bind to the complex by mutual reactions such as an "antigen"-"antibody" reaction, a "glycan"-"protein" reaction, a "glycan"-"lectin" reaction, a "protein"-"peptide chain" reaction, a "protein"-"protein" reaction, and a "protein"-"nucleotide chain" reaction.

The measurement method may be performed according to a measurement method of a sandwich method described in a document [for example, "Enzyme Immunoassay, 2nd Edition" (edited by Eiji Ishikawa, Igaku shoin) 1982)] usually referred to in the field.

In the sandwich method, for example, a first binding substance that recognizes the analyte is immobilized on a surface of a solid-phase carrier such as magnetic silica particles, a sample containing the analyte, the solid-phase carrier, and a second binding substance that recognizes the analyte and is labeled with peroxidase are mixed, and the immobilized first binding substance, the analyte in the sample, and the second binding substance are brought into contact with each other. As a result, a peroxidase-labeled complex, which is a complex of the immobilized first binding substance, the analyte in the sample, and the second binding substance labeled with peroxidase, is formed. Next, the solid-phase carrier carrying the peroxidase-labeled complex is separated into a B/F fraction, the amount of peroxidase in the peroxidase-labeled complex is measured, and the amount of the analyte in the sample is measured based on the amount of peroxidase in the peroxidase-labeled complex.

Alternatively, for example, a first binding substance that recognizes the analyte is immobilized on a surface of a solid-phase carrier such as magnetic silica particles, a sample containing the analyte, the solid-phase carrier, and "a third binding substance that recognizes a complex of the analyte and a first binding substance that recognizes the analyte", the third binding substance being labeled with peroxidase, are mixed, and the immobilized first binding substance, the analyte in the sample, and the third binding substance are brought into contact with each other. As a result, a peroxidase-labeled complex, which is a complex of the immobilized first binding substance, the analyte in the sample, and the third binding substance labeled with peroxidase, is formed. Next, the solid-phase carrier carrying the peroxidase-labeled complex is separated into a B/F fraction, the amount of peroxidase in the peroxidase-labeled complex is measured, and the amount of the analyte in the sample is measured based on the amount of peroxidase in the peroxidase-labeled complex.

As the solid-phase carrier, any support (particularly, an insoluble support) used in a normal immunological assay method can be used. Examples thereof include organic substances such as polystyrene, polyacrylic acid, polymethacrylic acid, polymethyl methacrylate, polyacrylamide, polyglycidyl methacrylate, polypropylene, polyolefin, polyimide, polyurethane, polyester, polyvinyl chloride, polyethylene, polychlorocarbonate, silicone resin, silicone rubber, agarose, dextran, and an ethylene-maleic acid anhydride copolymer; inorganic substances such as glass, silicon oxide, diatomaceous earth, porous glass, sintered glass, alumina, silica gel, and a metal oxide; magnetic materials such as iron, cobalt, nickel, magnetite, and chromite; and alloys of these magnetic materials; and materials prepared from these magnetic materials.

The immobilization can be performed on a solid phase (for example, any surface such as beads, magnetic beads, a membrane, and a plate). Various commercially available magnetic beads can be used. As the magnetic beads, for example, beads disclosed in WO2012/173002A may be used.

Specifically, for example, the analyte in the sample and the first binding substance that recognizes the analyte and is immobilized on a surface of a solid-phase carrier such as magnetic silica particles are brought into contact with each other to form a complex of the first binding substance that recognizes the analyte and is immobilized on the surface of the solid-phase carrier and the analyte in the sample. Next, the complex is brought into contact with the second binding substance labeled with peroxidase to form a complex (peroxidase-labeled complex) of the first binding substance that recognizes the analyte and is immobilized on the solid-phase carrier, the analyte in the sample, and the second binding substance labeled with peroxidase. The peroxidase-labeled complex is separated into a B/F fraction, the amount of peroxidase in the peroxidase-labeled complex is measured, and the amount of the analyte in the sample can be measured based on the amount of peroxidase in the peroxidase-labeled complex.

In the above-described method, the second binding substance labeled with peroxidase is reacted after the analyte in the sample and the first binding substance that recognizes the immobilized measurement target substance are reacted thereto. However, the immobilized first binding substance that recognizes the analyte may be reacted after the analyte in the sample and the second binding substance labeled with peroxidase are reacted, or all of these three may be reacted at the same time.

The B/F separation (bound/free separation) in the above-described sandwich method means separation of a substance carried on the solid-phase carrier and other substances. That is, the B/F separation in the present invention is
(1) separation of "a complex of the first binding substance that recognizes the analyte and is immobilized on the surface of the solid-phase carrier and the analyte in the sample", and an analyte or other substances in the reaction system that do not participate in the formation of the complex, and
(2) separation of a peroxidase-labeled complex and a second binding substance labeled with peroxidase or other substances in the reaction system that do not participate in the formation of the peroxidase-labeled complex.

The sandwich method can also be performed by a microfluidic chip (micro-total analysis system; µTAS). The microfluidic chip includes a flow path, and the flow path has a sample introduction port. For example, at the sample introduction port, the first binding substance that recognizes the analyte and is bound to a polycation or a polyanion (for example, DNA) is brought into contact with the analyte in the sample to form a complex of the first binding substance that recognizes the analyte and the analyte. Next, the complex is moved by applying an electric field, and in a case where the second binding substance labeled with peroxidase and the complex, which are present in the middle of the flow path, come into contact with each other, a complex (peroxidase-labeled complex) of the first binding substance, the analyte, and the second binding substance labeled with peroxidase is formed. Furthermore, the peroxidase-labeled complex is moved in the flow path by the electric field, and the peroxidase-labeled complex is B/F-separated and reaches a detection unit on the flow path. In the detection unit, the amount of peroxidase in the peroxidase-labeled complex is measured, and the amount of the analyte in the sample may be measured based on the amount of peroxidase in the peroxidase-labeled complex.

In the measurement method according to the embodiment of the present invention, the analyte in the sample and the first binding substance that recognizes the analyte and is immobilized on the surface of the solid-phase carrier may be brought into contact with each other by a usually performed treatment such as stirring and mixing. The reaction time may be appropriately set depending on the analyte and the first binding substance, but is usually 1 minute to 24 hours, preferably 1 minute to 1 hour, more preferably 1 to 10 minutes, and particularly preferably 1 to 5 minutes.

The B/F separation in the measurement method according to the embodiment of the present invention is performed, for example, by using the magnetism of the solid-phase carrier, collecting the solid-phase carrier from the outside of the reactor or the like with a magnet or the like, discharging the reaction solution, adding a washing solution, removing the magnet, mixing and dispersing the solid-phase carrier, and washing the solid-phase carrier. The above-described operation may be repeated 1 to 3 times. The washing solution is not particularly limited as long as it is usually used in the field.

As the peroxidase, horseradish peroxidase (HRP), microperoxidase, or the like can be used.

To bond the peroxidase to the second binding substance or the third binding substance, a method usually used in the field, for example, a known labeling method generally performed in a known EIA [for example, Ikagaku Jikken Koza, Vol. 8, supervised by Yuichi Yamamura, 1st ed., Nakayama Shoten, 1971; Zusetsu Keiko Kotai, Akira Kawao, 1st ed., Soft Science-sha, 1983; Koso Meneki Sokuteiho, edited by Eiji Ishikawa, Tadashi Kawai, and Kiyoshi Miyai, 2nd ed., Igaku-Shoin, 1982; and the like] may be used.

As the amount of the peroxidase used, it is preferable that the second binding substance or the third binding substance and the peroxidase are used, for example, in a molar ratio of usually 1:0.5 to 20, preferably 1:1 to 15, and more preferably 1:1 to 10. In addition, the binding substance that recognizes the analyte labeled with peroxidase may be used by being contained in a buffer solution usually used in the field, such as a Tris buffer solution, a phosphate buffer solution, a veronal buffer solution, a boric acid buffer solution, and a Good's buffer solution (for example, a MES (2-morpholinoethanesulfonic acid) buffer solution or the like), or the like.

The concentration of the peroxidase to which the second binding substance or the third binding substance is bonded is not particularly limited, but is preferably 0.1 nmol/L or more and 5,000 nmol/L or less, more preferably 1 nmol/L or more and 500 nmol/L or less, still more preferably 3 nmol/L or more and 300 nmol/L or less, and particularly preferably 5 nmol/L or more and 150 nmol/L or less.

In a case where an antibody is used as the binding substance, the pH of the above-described buffer solution need only be in a range that does not suppress the antigen-antibody reaction, and is usually 5 to 9. In addition, the buffer solution may contain, for example, a stabilizer such as albumin, globulin, water-soluble gelatin, and polyethylene glycol, a surfactant, and sugars, as long as the antigen-antibody reaction is not inhibited. It is noted that in the present specification, the pH means a numerical value measured in accordance with JIS K0400-12-10:2000 (measurement temperature: 25°C).

### <Measurement kit>

According to the present invention, it is possible to provide a measurement kit including: a peroxidase to which a binding substance that recognizes an analyte is bound; a chemiluminescent substance; an oxidizing agent; and the enhancer reagent for a chemiluminescence reaction according to the embodiment of the present invention.

According to the present invention, it is further possible to provide a measurement kit including: a peroxidase to which a binding substance that recognizes a complex of the analyte and a binding substance that recognizes the analyte is bound; a chemiluminescent substance; an oxidizing agent; and the enhancer reagent for a chemiluminescence reaction according to the embodiment of the present invention.

The preferred aspects and specific examples of each of the peroxidase to which the binding substance that recognizes the analyte is bound, the peroxidase to which the binding substance that recognizes the complex of the analyte and the binding substance that recognizes the analyte is bound, the chemiluminescent substance, the oxidizing agent, and the enhancer reagent for a chemiluminescence reaction according to the embodiment of the present invention are as described above in the present specification. In addition, the peroxidase to which the binding substance that recognizes the analyte is bound, the peroxidase to which the binding substance that recognizes the complex of the analyte and the binding substance that recognizes the analyte is bound, the chemiluminescent substance, the oxidizing agent, and the enhancer reagent for a chemiluminescence reaction according to the embodiment of the present invention in the present measurement kit may be contained in the reagent such that the concentration thereof is as described above in the present specification at the time of reaction.

In the measurement kit according to the embodiment of the present invention, the concentration (concentration as a reagent) of the chemiluminescent substance is not particularly limited, but is preferably 0.2 mmol/L or more and 20 mmol/L or less, more preferably 0.4 mmol/L or more and 15.0 mmol/L or less, still more preferably 0.5 mmol/L or more and 10.0 mmol/L or less, and particularly preferably 0.5 mmol/L or more and 5.0 mmol/L or less.

In the measurement kit according to the embodiment of the present invention, the concentration (concentration as a reagent) of the oxidizing agent is not particularly limited, but is preferably 1.0 mmol/L or more and 40 mmol/L or less, more preferably 2 mmol/L or more and 20 mmol/L or less, and still more preferably 2.8 mmol/L or more and 7.0 mmol/L or less.

In the measurement kit according to the embodiment of the present invention, the concentration (concentration as a reagent) of the one or more compounds represented by Formula (1) is not particularly limited, but is preferably 20 µmol/L or more and 1,000 µmol/L or less, more preferably 100 µmol/L or more and 1,000 µmol/L or less, still more preferably 150 µmol/L or more and 800 µmol/L or less, and particularly preferably 200 µmol/L or more and 600 µmol/L or less.

In the measurement kit according to the embodiment of the present invention, the concentration (concentration as a reagent) of the peroxidase to which the second binding substance or the third binding substance is bound is not particularly limited, but is preferably 0.1 nmol/L or more and 5,000 nmol/L or less, more preferably 1 nmol/L or more and 500 nmol/L or less, still more preferably 3 nmol/L or more and 300 nmol/L or less, and particularly preferably 5 nmol/L or more and 150 nmol/L or less.

The measurement kit according to the embodiment of the present invention may further include another binding substance that recognizes the analyte, which is different from the binding substance bound to the peroxidase, or a solid-phase carrier on which the binding substance is immobilized.

The present invention will be more specifically described based on the following examples, but the present invention is not limited to the examples.

### Examples

### <Synthesis example> Synthesis of various compounds

The purification by silica gel column chromatography was performed using an automatic purification device ISOLERA (manufactured by Biotage).

As a carrier in normal phase chromatography, CHROMATOREX Q-PACK (manufactured by Fuji Silysia Chemical Ltd.) was used.

As a carrier in reverse phase chromatography, Sfar C18 (manufactured by Biotage) was used.

Mass (MS) spectrum was measured using ACQUITY SQD LC/MS System (manufactured by Waters Corporation), ionization method: electrospray ionization (ESI) method was used to measure.

Retention time (RT) was measured using SQD (Waters Corporation) and shown in minutes (min).
Column: BEH C18 1.7 µm, 2.1 x 30 mm (manufactured by Waters Corporation)
Solvent: Liquid A: 0.1% formic acid-water
Liquid B: 0.1% formic acid-acetonitrile
Gradient cycle: 0.00 min (liquid A/liquid B = 95/5), 2.00 min (liquid A/liquid B = 5/95), 3.00 min (liquid A/liquid B = 95/5)
Flow rate: 0.5 mL/min
Column temperature: 40°C
Detection wavelength: 254 nm

### Synthesis of 4-(5-fluoro-6-methoxypyridin-3-yl)phenol

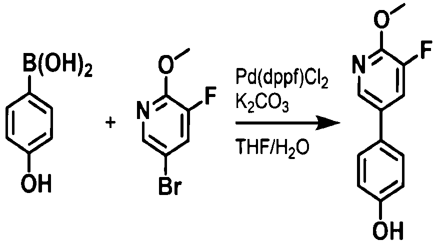

5-bromo-3-fluoro-2-methoxypyridine (239 mg), potassium carbonate (321 mg), water (160 µL), and 1,1-bis(diphenylphosphino)ferrocene dichloropalladium (II) (170 mg) were added to 4-hydroxyphenylboronic acid (160 mg), and the mixture was stirred at 120°C for 1 hour under a nitrogen atmosphere using Initiator^{TM} (Biotage). The reaction solution was purified by normal phase column chromatography (ethyl acetate/hexane = 0/100 → 70/30) and reverse phase column chromatography (acetonitrile/water = 0/100 → 100/0), thereby obtaining 4-(5-fluoro-6-methoxypyridin-3-yl)phenol as a white solid (48.0 mg).
MS (ESI m/z): (M+H) 220.4
RT (min): 1.26

The compounds shown in Table 1 were synthesized in the same manner as in the synthesis of 4-(5-fluoro-6-methoxypyridin-3-yl)phenol, except that 5-bromo-3-fluoro-2-methoxypyridine was changed to the raw material shown in Table 1.

**[Table 1]**

| Structure | Raw material name | Compound name | MS (ESI m/z) | RT/min |
|---|---|---|---|---|
| 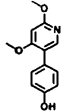 | 5-Bromo-2,4-dimethoxypyridine | 4-(4,6-Dimethoxypyridin-3-yl)phenol | 232.4 | 0.77 |
| 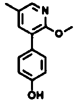 | 3-Bromo-2-methoxy-5-methylpyridine | 4-(2-Methoxy-5-methylpyridin-3-yl)phenol | 216.4 | 1.23 |
| 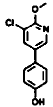 | 5-Bromo-3-chloro-2-methoxypyridine | 4-(5-Chloro-6-methoxypyridin-3-yl)phenol | 236.4 | 1.38 |
| 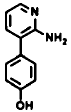 | 3-Bromopyridine-2-amine | 4-(2-Aminopyridin-3-yl)phenol | 187.4 | 0.46 |
| 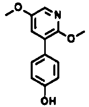 | 3-Bromo-2,5-dimethoxypyridine | 4-(2,5-Dimethoxypyridin-3-yl)phenol | 232.4 | 1.17 |
| 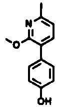 | 3-Bromo-2-methoxy-6-methylpyridine | 4-(2-Methoxy-6-methylpyridin-3-yl)phenol | 216.4 | 1.34 |
| 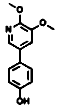 | 5-Bromo-2,3-dimethoxypyridine | 4-(5,6-Dimethoxypyridin-3-yl)phenol | 232.4 | 1.05 |
| 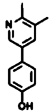 | 5-Bromo-2,3-dimethylpyridine | 4-(5,6-Dimethylpyridin-3-yl)phenol | 200.4 | 0.57 |
| 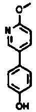 | 5-Bromo-2-methoxypyridine | 4-(6-Methoxypyridin-3-yl)phenol | 202.3 | 1.07 |
| 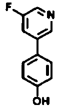 | 3-Bromo-5-fluoropyridine | 4-(5-Fluoropyridin-3-yl)phenol | 190.3 | 0.96 |
| 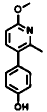 | 3-Bromo-6-methoxy-2-methylpyridine | 4-(6-Methoxy-2-methylpyridin-3-yl)phenol | 216.4 | 0.95 |
| 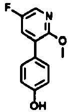 | 3-Bromo-5-fluoro-2-methoxypyridine | 4-(5-Fluoro-2-methoxypyridin-3-yl)phenol | 220.4 | 1.28 |
| 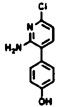 | 3-Bromo-6-chloropyridine-2-amine | 4-(2-Amino-6-chloropyridin-3-yl)phenol | 221.3 | 1.11 |
| 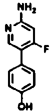 | 5-Bromo-4-fluoropyridine-2-amine | 4-(6-Amino-4-fluoropyridin-3-yl)phenol | 205.3 | 0.52 |
| 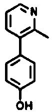 | 3-Bromo-2-methylpyridine | 4-(2-methylpyridin-3-yl)phenol | 186.3 | 0.45 |
| 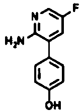 | 3-Bromo-5-fluoropyridine-2-amine | 4-(2-Amino-5-fluoropyridin-3-yl)phenol | 205.3 | 0.58 |
| 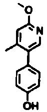 | 5-Bromo-2-methoxy-4-methylpyridine | 4-(6-Methoxy-4-methylpyridin-3-yl)phenol | 216.4 | 0.53 |
| 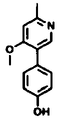 | 5-Bromo-4-methoxy-2-methylpyridine | 4-(4-Methoxy-6-methylpyridin-3-yl)phenol | 216.4 | 0.53 |
| 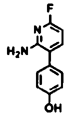 | 3-Bromo-6-fluoropyridine-2-amine | 4-(2-Amino-6-fluoropyridin-3-yl)phenol | 205.3 | 1.01 |
| 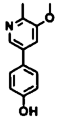 | 5-Bromo-3-methoxy-2-methylpyridine | 4-(5-Methoxy-6-methylpyridin-3-yl)phenol | 216.4 | 0.60 |
| 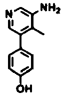 | 5-Bromo-4-methylpyridine-3-amine | 4-(5-Amino-4-methylpyridin-3-yl)phenol | 201.4 | 0.50 |
| 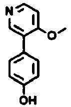 | 3-Bromo-4-methoxypyridine | 4-(4-Methoxypyridin-3-yl)phenol | 202.4 | 0.49 |
| 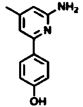 | 6-Bromo-4-methylpyridine-2-amine | 4-(6-Amino-4-methylpyridin-2-yl)phenol | 201.4 | 0.63 |
| 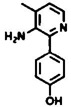 | 2-Bromo-4-methylpyridine-3-amine | 4-(3-Amino-4-methylpyridin-2-yl)phenol | 201.4 | 0.44 |
| 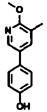 | 5-Bromo-2-methoxy-3-methylpyridine | 4-(6-Methoxy-5-methylpyridin-3-yl)phenol | 216.4 | 1.30 |

### Synthesis of 4-(6-methoxypyridin-2-yl)phenol

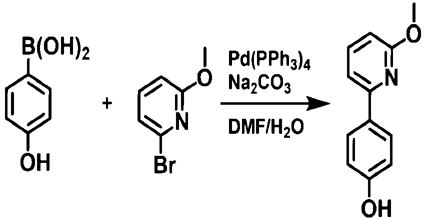

A solution of 2-bromo-6-methoxypyridine (136 mg) in N,N-dimethylformamide (1.0 mL), sodium carbonate (154 mg), water (100 µL), and tetrakis(triphenylphosphine)palladium (168 mg) were added to 4-hydroxyphenylboronic acid (100 mg), and the mixture was stirred at 120°C for 1 hour under a nitrogen atmosphere using Initiator^{TM} (Biotage). The reaction solution was purified by normal phase column chromatography (ethyl acetate/hexane = 0/100 → 100/0) and reverse phase column chromatography (0.1% formic acid-acetonitrile/0.1% formic acid-water = 0/100 → 100/0), thereby obtaining 4-(6-methoxypyridin-2-yl)phenol as a white solid (31.6 mg).
MS (ESI m/z): 202.4 (M-H)
RT (min): 0.48

The compounds shown in Table 2 were synthesized in the same manner as in the synthesis of 4-(6-methoxypyridin-2-yl)phenol, except that 2-bromo-6-methoxypyridine was changed to the raw material shown in Table 1.

**[Table 2]**

| Structure | Raw material name | Compound name | MS (ESI m/z) | RT/min |
|---|---|---|---|---|
| 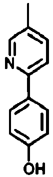 | 2-Bromo-5-methylpyridine | 4-(5-methylpyridin-2-yl)phenol | 186.4 | 0.54 |
| 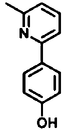 | 2-Bromo-6-methylpyridine | 4-(6-methylpyridin-2-yl)phenol | 186.4 | 0.49 |
| 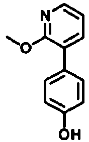 | 3-Bromo-2-methoxypyridine | 4-(2-Methoxypyridin-3-yl)phenol | 202.4 | 1.11 |
| 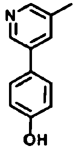 | 3-Bromo-5-methylpyridine | 4-(5-methylpyridin-3-yl)phenol | 186.4 | 0.54 |

### <Examples 1 to 29> Evaluation of each of enhancers in measurement of human chorionic gonadotropin (hCG)

### 1. Preparation of luminescent reagent (hCG)

A constitutional reagent required for the measurement was prepared using the following reagent raw materials.

MES (2-morpholinoethanesulfonic acid monohydrate) (manufactured by Dojindo Laboratories), sodium chloride (manufactured by FUJIFILM Wako Pure Chemical Corporation), BCN300S (manufactured by Nitta Gelatin Inc.), Block Ace (manufactured by KAC Co., Ltd.), BSA (bovine serum albumin) (manufactured by Sigma-Aldrich Japan G.K.), sodium orthovanadate (manufactured by Sigma-Aldrich Japan G.K.), sodium luminol salt (manufactured by FUJIFILM Wako Pure Chemical Corporation), phosphoric acid (manufactured by FUJIFILM Wako Pure Chemical Corporation), EDTA-2Na (manufactured by Dojindo Laboratories), hydrogen peroxide (manufactured by FUJIFILM Wako Pure Chemical Corporation), and dipotassium hydrogen phosphate (manufactured by FUJIFILM Wako Pure Chemical Corporation).

### (i) "First reagent" magnetic particle-immobilized antibody-containing reagent

3-Aminopropyltriethoxysilane (manufactured by Tokyo Chemical Industry Co., Ltd.) and succinic anhydride (manufactured by FUJIFILM Wako Pure Chemical Corporation) were reacted with Magrapid MGP-010T (manufactured by Sanyo Chemical Industries, Ltd.) in this order, and the mixture was collected with a neodymium magnet and the supernatant was removed to obtain magnetic particles having a carboxyl group. Next, the carboxyl group was activated by commercially available N-hydroxysuccinimide (manufactured by FUJIFILM Wako Pure Chemical Corporation) and water-soluble carbodiimide (WSC) (manufactured by Dojindo Laboratories), and then Mab to hCG beta (manufactured by Meridian Japan Co., Ltd.), which is an anti-hCG antibody, was reacted therewith at 26°C for 12 to 16 hours, and the magnetic particles were collected with a neodymium magnet and the supernatant was removed to prepare magnetic particles immobilized with an anti-hCG antibody, thereby preparing a first reagent consisting of the following composition.

"First reagent": 0.75 mg/mL anti-hCG antibody-immobilized magnetic particle, 50 mM MES (pH 5.5), 500 mM sodium chloride, 3.0% BCN300S

### (ii) "Second reagent" (reaction buffer solution): 50 mM 3-morpholinopropanesulfonic acid (MOPS) (manufactured by Dojindo Laboratories) (pH 7.5), 500 mM sodium chloride, 0.4% Block Ace

### (iii) "Third reagent" labeled antibody-containing reagent

MAB <HCG> M-INN22 IgG (manufactured by F. Hoffmann-La Roche Ltd.), which is an anti-hCG antibody, was digested with pepsin, and then F(ab')2 was separated by a column (diameter: 1.5 cm × length: approximately 95 cm) filled with Sephacryl S-200HR (manufactured by Cytiva). The obtained F(ab')2 was reduced with cysteamine hydrochloride (manufactured by Sigma-Aldrich Japan Co., Ltd.), and then Fab' was separated by a column (diameter: 1.5 cm × length: approximately 40 cm) filled with G-25 Superfine (manufactured by Cytiva). On the other hand, peroxidase (POD) (manufactured by F. Hoffmann-La Roche Ltd.) was maleimided using a maleimide reagent Sulfo-KMUS (manufactured by Dojindo Laboratories), and the reaction mixture was purified by a Sephadex G-25 column to remove unreacted Sulfo-KMUS, thereby obtaining maleimide-modified POD. The prepared Fab' and the maleimide-modified POD were mixed and separated by a Sephacryl S-100HR column to prepare a POD-labeled anti-hCG antibody. Using this, a third reagent having the following composition was prepared.

"Third reagent": 16 nmol/L POD-labeled anti-hCG antibody, 50 mM MES (pH 6.5), 150 mM sodium chloride, 2.0% BSA

### (iv) "Fourth reagent"

1.295 g (50 mM) of TAPSO (manufactured by FUJIFILM Wako Pure Chemical Corporation), 0.09 g (0.09%) of boric acid (manufactured by FUJIFILM Wako Pure Chemical Corporation), 0.28 mL (0.280%) of 5 mol/L NaOH (manufactured by FUJIFILM Wako Pure Chemical Corporation), and 0.1555 g (0.5 mM) of L-012 (manufactured by FUJIFILM Wako Pure Chemical Corporation) were dissolved in 100 mL of purified water, and each of the synthesized compounds was dissolved such that the concentration was 0.2 mM, thereby preparing a fourth reagent.

### (v) "Fifth reagent"

"Fifth reagent": 68 µL/L phosphoric acid (manufactured by FUJIFILM Wako Pure Chemical Corporation), 4 mM EDTA-2Na (manufactured by Dojindo Laboratories), 335 µL/L hydrogen peroxide (manufactured by FUJIFILM Wako Pure Chemical Corporation)

### 2. Preparation of blank sample

10.46 g (50 mM) of bis(2-hydroxyethyl)iminotris(hydroxymethyl)methane (manufactured by FUJIFILM Wako Pure Chemical Corporation), 1.7 mL (0.17%) of 5N-HCl (manufactured by FUJIFILM Wako Pure Chemical Corporation), and 20 g (2%) of bovine serum albumin (manufactured by Sigma-Aldrich Japan Co., Ltd.) were dissolved in 1,000 mL of purified water to prepare a blank sample.

### 3. Preparation of measurement sample (hCG)

Human Chorionic Gonadotropin (manufactured by Meridian Japan Co., Ltd.) was dissolved in 10 mL of the blank sample prepared in "2. Preparation of blank sample" to prepare a high-unit hCG solution of 20,000 mIU/mL. Using the blank sample manufactured in "2. Preparation of blank sample" and the high-unit hCG solution, 0, 1, 3, 7,000, and 10,000 mIU/mL hCG solutions were prepared as measurement samples.

### 4. Evaluation of luminescent reagent

Using the first to fifth reagents prepared in "1. Preparation of luminescent reagent (hCG)" and the AcuRapid B/F separation solution (manufactured by FUJIFILM Wako Pure Chemical Corporation), the luminescence intensity of each solution was measured by an automatic chemiluminescence enzyme immunoassay device AcuRapid (manufactured by FUJIFILM Wako Pure Chemical Corporation) according to the following procedure. 50 µL of the first reagent added to the reaction cuvette was heated at 67°C for 20 seconds while magnetic particles were collected using a neodymium magnet, and the supernatant was removed. Subsequently, 140 µL of the second reagent and 10 µL of the measurement samples (hCG solutions) at concentrations of 0, 1, 3, 7000, and 10,000 mIU/mL were added thereto and stirred, and the mixture was heated at 37°C for 3 minutes. After completion of the heating, the magnetic particles were collected using a neodymium magnet, a reagent other than the magnetic particles was removed, and the magnetic particles were washed three times with a washing solution. Subsequently, 50 µL of the third reagent was added, and the mixture was heated at 37°C for 3 minutes. After the heating was completed, the mixture was collected with a neodymium magnet to remove the reagents other than the magnetic particles, and washed with a washing solution three times. After the washing was completed, 100 µL of the fourth reagent and 100 µL of the fifth reagent were added thereto, and the mixture was reacted at 37°C for 20 seconds, and the luminescence intensities were measured.

The sensitivity and the high-end slope were calculated from the obtained luminescence intensities. The sensitivity was calculated from (luminescence intensity of 3 mIU/mL hCG solution)/(luminescence intensity of blank sample), and the high-end slope was calculated from (luminescence intensity of 10,000 mIU/mL hCG solution)/(luminescence intensity of 7,000 mIU/mL hCG solution).

### <Comparative Example 1> Evaluation of PPP in measurement of hCG

The luminescent reagent was prepared and evaluated by the same method as in Examples 1 to 29, except for the following.

As the "fourth reagent", a solution in which PPP was dissolved such that the concentration was 0.2 mM instead of each of the synthesized compounds in (iv) "Fourth reagent" of Example 1 was used.

**[Table 3]**

| Evaluation result in HCG | | | |
|---|---|---|---|
| Luminescent substrate: L-012 | Structure | Sensitivity | High-end slope |
| | | D: <6.5 | E: ≤1.21 |
| | | C: 6.5 to 8 | D: 1.22 to 1.23 |
| | | B: 8 to 9 | C: 1.24 to 1.25 |
| | | A: 9 to 10 | B: 1.26 to 1.27 |
| | | | A: ≥1.28 |
| Comparative Example 1: PPP | 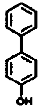 | D | E |
| Example 1 | 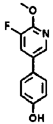 | A | A |
| Example 2 | 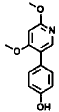 | B | A |
| Example 3 (use commercially available product) | 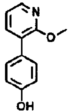 | B | A |
| Example 4 | 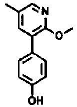 | B | A |
| Example 5 | 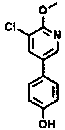 | C | A |
| Example 6 | 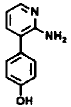 | C | B |
| Example 7 | 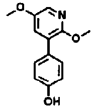 | B | A |
| Example 8 | 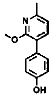 | B | A |
| Example 9 | 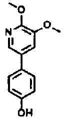 | C | A |
| Example 10 | 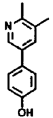 | C | B |
| Example 11 | 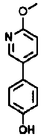 | C | A |
| Example 12 (use commercially available product) | 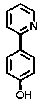 | C | C |
| Example 13 | 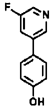 | C | A |
| Example 14 | 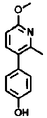 | C | A |
| Example 15 | 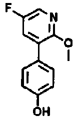 | C | A |
| Example 16 | 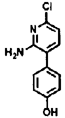 | B | C |
| Example 17 | 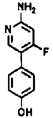 | C | C |
| Example 18 | 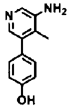 | D | A |
| Example 19 | 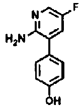 | D | A |
| Example 20 | 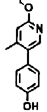 | D | A |
| Example 21 | 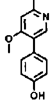 | D | B |
| Example 22 | 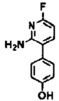 | D | A |
| Example 23 | 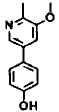 | D | A |
| Example 24 (use commercially available product) | 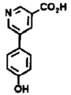 | D | A |
| Example 25 | 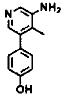 | D | A |
| Example 26 | 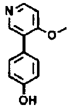 | D | A |
| Example 27 | 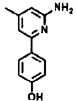 | D | B |
| Example 28 | 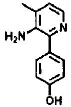 | D | A |
| Example 29 | 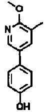 | D | A |

### <Examples 30 to 35> Evaluation of enhancer in measurement of estradiol (E2)

### 1. Preparation of luminescent reagent (E2)

A constitutional reagent required for the measurement was prepared using the following reagent raw materials.

MES (2-morpholinoethanesulfonic acid monohydrate) (manufactured by Dojindo Laboratories), sodium chloride (manufactured by FUJIFILM Wako Pure Chemical Corporation), BCN300S (manufactured by Nitta Gelatin Inc.), Block Ace (manufactured by KAC Co., Ltd.), BSA (bovine serum albumin) (manufactured by Sigma-Aldrich Japan G.K.), sodium orthovanadate (manufactured by Sigma-Aldrich Japan G.K.), sodium luminol salt (manufactured by FUJIFILM Wako Pure Chemical Corporation), phosphoric acid (manufactured by FUJIFILM Wako Pure Chemical Corporation), EDTA-2Na (manufactured by Dojindo Laboratories), hydrogen peroxide (manufactured by FUJIFILM Wako Pure Chemical Corporation), and dipotassium hydrogen phosphate (manufactured by FUJIFILM Wako Pure Chemical Corporation).

### (i) "First reagent" magnetic particle-immobilized antibody-containing reagent

3-Aminopropyltriethoxysilane (manufactured by Tokyo Chemical Industry Co., Ltd.) and succinic anhydride (manufactured by FUJIFILM Wako Pure Chemical Corporation) were reacted with Magrapid MGP-010T (manufactured by Sanyo Chemical Industries, Ltd.) in this order, and the mixture was collected with a neodymium magnet and the supernatant was removed to obtain magnetic particles having a carboxyl group. Next, the carboxyl group was activated by commercially available N-hydroxysuccinimide (manufactured by FUJIFILM Wako Pure Chemical Corporation) and water-soluble carbodiimide (WSC) (manufactured by Dojindo Laboratories), and then Sheep Monoclonal Antibodies (SMAs) to E2 5A11 (manufactured by BiOVENTIX), which is an anti-E2 antibody, was reacted at 26°C for 12 to 16 hours, and the magnetic particles were collected with a neodymium magnet and the supernatant was removed to prepare an anti-E2 antibody-immobilized magnetic particle, thereby preparing a first reagent consisting of the following composition.

"First reagent": 0.5 mg/mL of anti-E2 antibody-immobilized magnetic particle, 50 mM MES (pH 5.5), 500 mM sodium chloride, 3.0% BCN300S

### (ii) "Second reagent" (reaction buffer solution): 50 mM 3-morpholinopropanesulfonic acid (MOPS) (manufactured by Dojindo Laboratories) (pH 7.5), 500 mM sodium chloride, 0.4% Block Ace

### (iii) "Third reagent" labeled antibody-containing reagent

The complex of E2, which is an immunogen, and an anti-E2 antibody was injected into a mouse, which is an immunized animal, to immunize the mouse, and then lymphocytes were collected. The obtained lymphocytes and myeloma were fused with each other to screen for a hybridoma cell that produced a desired antibody. After cloning, a desired antibody-producing hybridoma was produced. The anti-immune complex M19-1G antibody produced by the hybridoma prepared by the above method was digested with pepsin, and then F(ab')2 was separated by a column (diameter: 1.5 cm × length: approximately 95 cm) filled with Sephacryl S-200HR (manufactured by Cytiva). The obtained F(ab')2 was reduced with cysteamine hydrochloride (manufactured by Sigma-Aldrich Japan Co., Ltd.), and then Fab' was separated by a column (diameter: 1.5 cm × length: approximately 40 cm) filled with G-25 Superfine (manufactured by Cytiva). On the other hand, peroxidase (POD) (manufactured by F. Hoffmann-La Roche Ltd.) was maleimided using a maleimide reagent Sulfo-KMUS (manufactured by Dojindo Laboratories), and the reaction mixture was purified by a Sephadex G-25 column to remove unreacted Sulfo-KMUS, thereby obtaining maleimide-modified POD. The prepared Fab' and the maleimide-POD were mixed and separated by a Sephacryl S-100HR column to prepare a POD-labeled anti-immune complex M19-1G antibody. Using this, a third reagent having the following composition was prepared.

"Third reagent": 6 nmol/L POD-labeled anti-immune complex M19-1G antibody, 50 mM MES (pH 6.5), 150 mM sodium chloride, 2.0% BSA

### (iv) "Fourth reagent"

1.82 g (50 mM) of 2-amino-2-hydroxymethyl-1,3-propanediol Tris(hydroxymethyl)aminomethane (manufactured by FUJIFILM Wako Pure Chemical Corporation), 0.690 mL (0.069%) of 5N-HCl (manufactured by FUJIFILM Wako Pure Chemical Corporation), 0.877 g (150 mM) of sodium chloride (manufactured by FUJIFILM Wako Pure Chemical Corporation), 8.57 mg (1 mM) of sodium orthovanadate (manufactured by Sigma-Aldrich Japan Co., Ltd.), 0.1 g (5 mM) of sodium luminol (manufactured by FUJIFILM Wako Pure Chemical Corporation), and each compound described in the table at the concentration of 0.2 mM were dissolved in 100 mL of purified water to prepare a fourth reagent.

### (v) "Fifth reagent"

"Fifth reagent": 68 µL/L phosphoric acid (manufactured by FUJIFILM Wako Pure Chemical Corporation), 4 mM EDTA-2Na (manufactured by Dojindo Laboratories), 335 µL/L hydrogen peroxide (manufactured by FUJIFILM Wako Pure Chemical Corporation)

### 2. Preparation of blank sample

10.46 g (50 mM) of bis(2-hydroxyethyl)iminotris(hydroxymethyl)methane (manufactured by FUJIFILM Wako Pure Chemical Corporation), 1.7 mL (0.17%) of 5N-HCl (manufactured by FUJIFILM Wako Pure Chemical Corporation), and 20 g (2%) of bovine serum albumin (manufactured by Sigma-Aldrich Japan Co., Ltd.) were dissolved in 1,000 mL of purified water to prepare a blank sample.

### 3. Preparation of measurement sample (E2)

β-Estradiol (manufactured by FUJIFILM Wako Pure Chemical Corporation) was dissolved in 10 mL of the blank sample prepared in "2. Preparation of blank sample" to prepare a high-unit E2 solution of 10,000 pg/mL. Using the blank sample and the high-unit E2 solution prepared in "2. Preparation of blank sample", 0, 200, 2,000, and 3,000 pg/mL E2 solutions were prepared as measurement samples.

### 4. Evaluation of luminescent reagent

The luminescence intensities were measured by the same method as in Example 1 using the first to fifth reagents and the AcuRapid B/F separation solution (manufactured by FUJIFILM Wako Pure Chemical Corporation), and the sensitivity and the high-end slope were calculated. The sensitivity was calculated from (luminescence intensity of 200 pg/mL E2 solution)/(luminescence intensity of blank), and the high-end slope was calculated from (luminescence intensity of 3,000 pg/mL E2 solution)/(luminescence intensity of 2,000 pg/mL E2 solution).

### <Comparative Example 2> Evaluation of TP in measurement of E2

The luminescent reagent was prepared and evaluated by the same method as in Examples 30 to 35, except for the following.

As the "fourth reagent", a solution in which TP was dissolved such that the concentration was 0.2 mM instead of each of the compounds described in (iv) "Fourth reagent" of Examples 30 to 35 was used.

**[Table 4]**

| Evaluation result in E2 | | | |
|---|---|---|---|
| Luminescent substrate: Luminol | Structure | Sensitivity | High-end slope |
| | | E: <35 | D: <1.0 |
| | | D: 35 to 40 | C: 1.0 to 1.3 |
| | | C: 40 to 45 | B: 1.3 to 1.6 |
| | | B: 45 to 50 | A: 1.6 to 2.0 |
| | | A: >50 | |
| Comparative Example 2: TP | 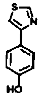 | E | D |
| Example 30 | 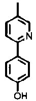 | A | B |
| Example 31 | 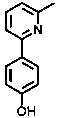 | C | A |
| Example 32 (use commercially available product) | 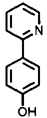 | C | A |
| Example 33 | 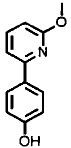 | E | A |
| Example 34 | 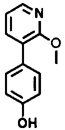 | C | B |
| Example 35 | 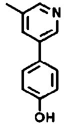 | D | A |

## Claims

1. An enhancer reagent for a chemiluminescence reaction, comprising:
a compound represented by Formula (1), 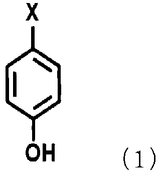
in the formula, X represents a pyridyl group which may be substituted with one or more groups selected from the group consisting of an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a silyl group, a silyloxy group, a hydroxyl group, a cyano group, a nitro group, a halogen atom, a carboxy group, a sulfo group, a phosphonyl group, a phosphoryl group, a boronic acid group, and a sulfanyl group.

2. The enhancer reagent according to claim 1,
wherein X is a 3-pyridyl group which may be substituted with one or more groups selected from the group consisting of an alkyl group, an alkenyl group, a cycloalkyl group, an aryl group, a heterocyclic group, an alkoxy group, a cycloalkoxy group, an aryloxy group, an alkoxycarbonyl group, a cycloalkoxycarbonyl group, an amino group, an acylamino group, a silyl group, a silyloxy group, a hydroxyl group, a cyano group, a nitro group, a halogen atom, a carboxy group, a sulfo group, a phosphonyl group, a phosphoryl group, a boronic acid group, and a sulfanyl group.

3. The enhancer reagent according to claim 1,
wherein X is a 3-pyridyl group which is substituted with one or more groups selected from the group consisting of an alkyl group, an alkoxy group, a halogen atom, and an amino group.

4. The enhancer reagent according to claim 1,
wherein X is a 3-pyridyl group which is substituted with one or more groups selected from the group consisting of an alkyl group having 1 to 5 carbon atoms, an alkoxy group having 1 to 5 carbon atoms, and a fluorine atom.

5. The enhancer reagent according to claim 1,
wherein the compound represented by Formula (1) is any one of the following compounds,

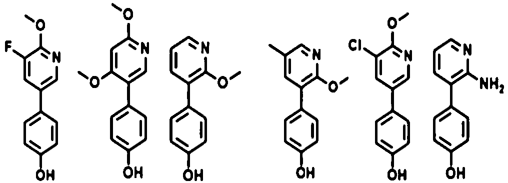

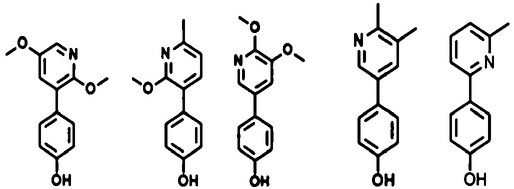

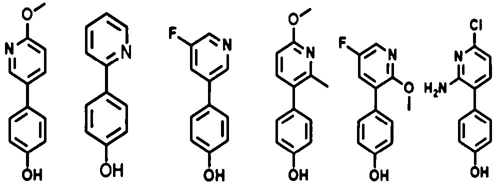

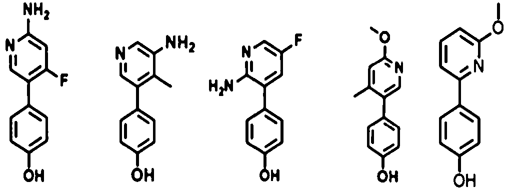

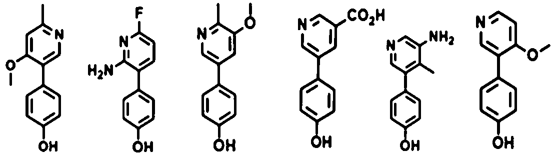

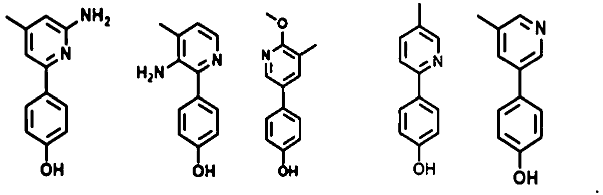

6. A compound represented by any one of the following formulae,
